# NOUVEAU FASCICULE DE BREVET EUROPEEN

(11) **EP 1 035 914 B2**
(45) Date de publication et mention de la décision concernant l'opposition: **04.05.2016**
(45) Mention de la délivrance du brevet: 09.01.2013
(21) Numéro de dépôt: 98962385.5
(22) Date de dépôt: 18.11.1998
(51) Int. Cl.: B01J 29/04, B01J 37/00, C07D 301/12

(54) **PROCEDE DE FABRICATION D'UN CATALYSEUR D'EPOXYDATION**
HERSTELLUNGSVERFAHREN EINES EPOXIDIERUNGSKATALYSATORS
PRODUCTION OF AN EPOXIDATION CATALYST

(30) Priorité: 27.11.1997 BE 9700962
(43) Date de publication de la demande: 20.09.2000
(73) Titulaire: SOLVAY SA, 1120 Bruxelles (BE)
(72) Inventeur: STREBELLE, Michel, B-1150 Bruxelles (BE); CATINAT, Jean-Pierre, B-7131 Waudrez (BE)
(74) Mandataire: Vande Gucht, Anne
(86) Numéro de dépôt international: PCT/EP1998/007527
(87) Numéro de publication internationale: WO 1999/028029

(56) Documents cités:
- EP-A- 0 326 759
- EP-A- 0 376 453
- EP-A- 0 568 336
- EP-A- 0 659 685
- EP-A- 0 706 824
- EP-A2- 0 712 662
- W. GERHARTZ ET AL.: 'Ullmann's Encyclopedia of Industrial Chemistry', vol. A5, 1996, VCH, WEINHEIM pages 352 - 353
- B. ELVERS ET AL.: 'Ullmann's Encyclopedia of Industrial Chemistry', vol. A28, 1996, VCH, WEINHEIM page 475,491

## Description

La présente invention concerne un procédé de fabrication des catalyseurs d'époxydation, en particulier des catalyseurs à base de zéolite au titane.

Il est connu d'utiliser des catalyseurs à base de silicalite au titane dans des réactions d'époxydation. Par exemple, dans la demande de brevet EP-A2-0 200 260 on utilise des microsphères à base de silicalite au titane de diamètre d'environ 20 µm obtenues par atomisation dans des réactions d'époxydation. Ce catalyseur connu donne lieu à un phénomène de désactivation. Des cycles de régénération, impliquant des manipulations, sont donc nécessaires. Lorsque ces catalyseurs de diamètre relativement faible sont utilisés dans des réactions d'époxydation, ils sont difficiles à isoler du milieu réactionnel pour pouvoir les transférer dans un traitement de régénération. US-A-6-106797 décrit des catalyseurs d'époxydation des olefines, d'oxidation pour la production de peroxide d'hydrogène et la synthèse d'hydroxy-lamine.

US-A-6106797 décrit des catalyseurs d'époxydation des oléfines, d'oxydation pour la production de péroxide d'hydrogène et la synthèse d'hydroxylamine.

La présente invention vise à remédier à ce problème en fournissant un procédé de fabrication d'un catalyseur facile à séparer du milieu de réaction d'époxydation en vue de le transporter dans une unité de régénération. Un autre objectif de l'invention est de fournir un catalyseur d'époxydation qui présente une bonne résistance mécanique, une activité catalytique élevée et une sélectivité élevée. Encore un autre objectif de l'invention est de fournir un procédé de fabrication d'un catalyseur facilement utilisable en lit fixe ou agité.

La présente invention concerne dès lors un procédé de fabrication d'un catalyseur d'époxydation à base de zéolite au titane qui se présente sous la forme de granules extrudés. Il a été constaté qu'un tel catalyseur présente simultanément les avantages suivants :
- il est facile à séparer du milieu de réaction d'époxydation en vue de le transporter dans une unité de régénération,
- il présente une bonne résistance mécanique, une activité catalytique élevée et une sélectivité élevée, et
- il est facilement utilisable en lit fixe ou agité.

Par zéolite au titane on entend désigner un solide contenant de la silice qui présente une structure cristalline microporeuse de type zéolite et dans laquelle plusieurs atomes de silicium sont remplacés par des atomes de titane.

L'objet de la présent invention est décrit dans les libellés des revendications 1 à 12.

La zéolite au titane présente avantageusement une structure cristalline de type ZSM-5, ZSM-11 ou MCM-41. Elle peut aussi présenter une structure cristalline de type zéolite β exempte d'aluminium. Elle présente de préférence une bande d'absorption infrarouge à environ 950 - 960 cm⁻¹. Les zéolites au titane de type silicalite conviennent bien. Celles répondant à la formule xTiO₂(1-x)SiO₂ dans laquelle x est de 0,0001 à 0,5, de préférence de 0,001 à 0,05, sont performantes. Des matériaux de ce type, connus sous le nom de TS-1, présentent une structure zéolitique cristalline microporeuse analogue à celle de la zéolite ZSM-5. Les propriétés et les principales applications de ces composés sont connues (B. Notari; Structure-Activity and Selectivity Relationship in Heterogeneous Catalysis; R.K. Grasselli and A.W. Sleight Editors; Elsevier; 1991; p. 243-256). Leur synthèse a été étudiée notamment par A. Van der Poel et J. Van Hooff (Applied Catalysis A; 1992; Volume 92, pages 93-111). D'autres matériaux de ce type ont une structure analogue à celle de la zéolite bêta ou de la zéolite ZSM-11.

Par granules extrudés on entend désigner des grains obtenus par extrusion. En particulier les granules sont obtenus en extrudant une masse extrudable contenant la zéolite au titane et en coupant l'extrudat sortant de l'extrudeuse en grains.

Les granules extrudés peuvent avoir une forme quelconque. Ils peuvent être pleins ou creux. Ils peuvent être de section ronde ou rectangulaire ou encore d'une autre section à surtace extérieure plus élevée. On préfère les formes cylindriques. Les granules extrudés de forme cylindrique ont avantageusement un diamètre d'au moins 0,5 mm, de préférence d'au moins 1 mm. Le diamètre est couramment d'au plus 5 mm, en particulierd'au plus 2 mm. Les formes cylindriques ont habituellement une longueur d'au moins 1 mm, en particulier d'au moins 2 mm. Les longueurs d'au plus 8 mm sont courantes, celles d'au plus 4 mm donnent de bons résultats. Les formes cylindriques ayant un diamètre de 0,5 à 5 mm, de préférence de 1 à 2 mm, et une longeur de 1 à 8 mm, de préférence de 2 à 4 mm conviennent bien.

La teneur en zéolite au titane dans le catalyseur est en général d'au moins 1 % en poids, en particulier d'au moins 50 % en poids. La teneur en zéolite au titane est le plus souvent d'au plus 99% en poids, en particulier d'au plus 98 % en poids. Le catalyseur contient généralement de 1 à 99 % en poids, de préférence de 50 à 98 % en poids, de zéolite au titane, le restant étant constitué d'une matrice. Cette matrice contient de préférence une matière siliceuse.

Le procédé selon l'invention comprenant les étapes (a) à (d) est décrit dans le libéllé de la revendication indépendante 1.

L'étape (a) consiste généralement à obtenir une pâte de viscosité telle que l'on puisse la mettre en oeuvre dans une extrudeuse. Le mélange peut être effectué dans un mélangeur ou un malaxeur quelconque. Tous les constituants du mélange peuvent être mélangés simultanément. En variante, le liant, le plastifiant, la substance porogène l'eau et, le cas échéant, les autres additifs peuvent être prémélangés avant d'y ajouter la poudre de zéolite au titane. Le mélange est avantageusement réalisé à température ambiante. En variante, le mélange peut être refroidi au cours de l'étape (a), par exemple à l'eau. La durée de l'étape (a) peut varier de 5 à 60 min.

La granulométrie de la poudre de zéolite au titane mise en oeuvre dans l'étape (a) peut varier dans une large mesure. Elle est de préférence caractérisée par un diamètre moyen inférieur ou égal à 10 µm, en particulier inférieur ou égal à 5 µm. Le diamètre moyen est généralement d'au moins 0,05 µm, en particulierd'au moins 0,1 µm. Des diamètres inférieurs à 0,05 µm conviennent également.

Le plastifiant utilisable dans l'étape (a) est un polysaccharide tel qu'un amidon ou une cellulose. Les celluloses conviennent bien. A titre d'exemples de cellulose on peut citer la méthyl-, carboxyméthyl- et hydroxyéthylcellulose. La méthylcellulose est préférée.

La quantité de plastifiant mise en oeuvre dans l'étape (a) peut varier dans une large mesure. Des quantités réduites d'au moins 1 % et inférieures à 10 % en poids par rapport au poids de zéolite au titane mis en oeuvre sont recommandées car elles conduisent à une meilleure résistance à l'attrition par rapport aux quantités plus élevées.

Le liant utilisable dans l'étape (a) est choisi parmi les siloxanes. On peut citer à titre d'exemples les éthers de méthyl- ou éthylsiloxane. Des résines siliconées à base de polyméthylsiloxane peuvent également être utilisées. Des résines siliconées de type polyméthyl/phénylsiloxane conviennent aussi. Il peut également s'agir de mélanges de différents oligomères de type méthylsiloxane. Le liant mis en oeuvre dans l'étape (a) peut être sous la forme d'une poudre. En variante, il peut être sous la forme d'une émulsion aqueuse. Il peut également être utilisé sous forme liquide. Les résines siliconées à base de polyméthylsiloxane sous forme d'une poudre et les mélanges de différents oligomères de type méthylsiloxane sous forme liquide sont préférés car ils conduisent à des catalyseurs de résistance mécanique plus élevée. Le liant est transformé, dans l'étape (d) de calcination, en une matière constituant la matrice présente dans le catalyseur selon l'invention.

La quantité de liant mise en oeuvre dans l'étape (a) varient de 5 à 20 % en poids par rapport au poids de zéolite au titane mis en oeuvre. Ces quantités conduisent à un meilleur compromis entre l'activité catalytique et la résistance mécanique par rapport aux quantités plus faibles et plus élevées.

Des lubrifiants peuvent également être ajoutés dans le mélange de l'étape (a). Il peut s'agirde composés à base de paraffine, de polyvinylpyrrolidone, de polyéthylèneoxyde et d'alcool polyvinylique.

Des substances porogènes sont ajoutées dans le mélange de l'étape (a). Ces substances sont éliminées lors de l'étape (d) de calcination et augmentent ainsi la porosité du catalyseur. La quantité de substance porogène mise en oeuvre est généralement d'au moins 5 % en poids, en particulier d'au moins 6 % en poids, par rapport au poids de zéolite au titane mis en oeuvre. Elle est habituellement d'au plus 35 % en poids, en particulier d'au plus 14 % en poids, par rapport au poids de zéolite au titane mis en oeuvre. Les quantités de 6 à 14 % en poids par rapport au poids de zéolite au titane mis en oeuvre conviennent particulièrement bien car elles conduisent à une meilleure résistance à l'attrition par rapport aux quantités plus élevées. La substance porogène est la mélamine.

L'étape (b) d'extrusion peut être réalisée dans une extrudeuse à piston. En variante, elle peut être réalisée dans une extrudeuse à vis.

L'étape (c) de séchage est avantageusement réalisée à des vitesses faibles de séchage pour assurer une bonne cohésion du catalyseur. Par exemple, un préséchage à basse température (par exemple de la température ambiante à 90 °C, éventuellement en combinaison avec une irradiation infrarouge ou de micro-ondes) peut d'abord être réalisé; ensuite, la température peut être montée lentement pour atteindre la température finale de séchage. En variante, lorsque l'eau peut être évacuée rapidement par une ventilation adéquate, la température peut être augmentée à une vitesse plus élevée. Typiquementon élève la température à une vitesse de 1 ° par minute. Le séchage est généralement effectué à une température finale d'au moins 400 °C. La température finale de séchage est habituellement d'au plus 500°C. Des températures plus basses de 100 à 400 °C peuvent convenir lorsque la durée du séchage est suffisamment longue, par exemple de 10 à 20 h.

L'étape (d) de calcination est généralement effectuée à une température d'au moins 300 °C, en particulier d'au moins 400 °C. La température est habituellement d'au plus 550°C, en particulier d'au plus 520 °C. Des températures qui dépassent 550 °C ne sont pas recommandées car la plupart des zéolites au titane ne résistent pas à de telles températures. La durée de l'étape (d) de calcination doit être suffisamment longue pour pouvoir éliminer la plus grande partie des résidus organiques provenant du liant et/ou du plastifiant. Des durées de 60 h sont typiques. Généralement, la durée est d'au moins 50 h et d'au plus 100 h. L'étape (d) de calcination est de préférence opérée sous atmosphère oxydante, par exemple sous air.

Le procédé comprenant les étapes (a) à (d) et une étape de granulation comme décrit ci-avant peut être utilisé pour fabriquer d'autres catalyseurs en forme de granules extrudés.

Le catalyseur produit selon le procédé de l'invention peut être utilisé dans la synthèse d'oxirannes par réaction entre un composé oléfinique avec un composé peroxyde.

L'oxiranne est de préférence le 1,2-époxy-3-chloropropane ou le 1,2-époxypropane. Le composé oléfinique est de préférence le chlorure d'allyle ou le propylène. Le composé peroxydé peut être choisi parmi ceux contenant de l'oxygène actif et capables d'effectuer une époxydation. Le peroxyde d'hydrogène et les composés peroxydés qui peuvent produire du peroxyde d'hydrogène dans les conditions de la réaction d'époxydation conviennent bien. Le composé peroxydé est de préférence le peroxyde d'hydrogène.

### Exemple (conforme à l'invention)

Dans cet exemple, des granules extrudés contenant du TS-1 ont d'abord été fabriqués. Ils ont ensuite été utilisés dans la synthèse d'épichlorhydrine (EPI) à partir de chlorure d'allyle (CAL) et de peroxyde d'hydrogène (H₂O₂). Une poudre de TS-1 a été mélangée avec :
- 15,8 g de liant (une poudre de résine siliconée de type polyméthylsiloxane avec une teneur en SiO₂ de 87 % après calcination à 500°C) pour 100 g de TS-1,
- 4 g de plastifiant (méthylcellulose de viscosité de 12000 mPas, la viscosité étant mesurée en solution aqueuse à 2 % en poids) pour 100 g de TS-1,
- 10 g de substance porogène (mélamine) pour 100 g de TS-1,
- 60 g d'eau pour 100 g de TS-1.

Le mélange a ensuite été malaxé à température ambiante pendant 25 min à une vitesse de rotation des cannes de 50 tours/min. La pâte obtenue a été introduite dans une extrudeuse munie d'une filière de 1 mm. L'extrudat a été séché à 120 °C pendant 15 h avant d'être calciné à 500 °C durant 60 h sous air avec un gradient de température de 1 ° par minute. L'extrudat séché et calciné a ensuite été découpé par une granulatrice à une longeur de 3 mm. Les granules obtenus contiennent 88 % en poids de TS-1 et 12 % de matrice siliceuse provenant de la calcination du liant.

Dans un réacteur boucle contenant un lit du catalyseur obtenu ci-avant (quantité de TS-1 introduite = 2 % en poids du milieu réactionnel) on a fait circuler un milieu réactionnel contenant du CAL, du méthanol et de l'H₂O₂ (à 35 %) dans des proportions molaires CAL/H₂O₂ = 2, méthanol/CAL = 7,8. Après 2,5 h de réaction à 25 °C, 89 % de la quantité d'H₂O₂ mise en oeuvre a été consommée. La sélectivité en EPI (le rapport molaire entre la quantité d'EPI produite et la somme des quantités de produits formés) était de 99 %.

## Revendications

1. Procédé de fabrication d'un catalyseur d'époxydation à base de zéolite au titane présentant une structure cristalline ZSM-5, ZSM-11, MCM-41, comprenant:
(a) une étape de malaxage d'un mélange comprenant une poudre de zéolite au titane, de l'eau, au moins un liant en une quantité de 5 % à 20 % en poids par rapport au poids de la zéolite au titane, ce liant étant choisi parmi les siloxanes, au moins un plastifiant choisi parmi les polysaccharides, au moins une substance porogène en une quantité de 5 à 35 % en poids par rapport au poids de zéolite au titane, et éventuellement d'autres additifs, pour former une pâte,
(b) une étape de mise en forme de la pâte obtenue dans l'étape (a) par extrusion, pour obtenir un extrudat,
(c) une étape de séchage, afin d'éliminer au moins une partie de l'eau,
(d) une étape de calcination, afin d'éliminer au moins une partie des résidus organiques présents, afin de transformer le liant en une matière constituant la matrice du catalyseur, et afin d'éliminer la substance porogène et d'augmenter ainsi la porosité du catalyseur,
et comprenant une étape de granulation effectuée entre l'étape (b) d'extrusion et l'étape (c) de séchage ou après l'étape (d) de calcination, afin d'obtenir des granules extrudés,
dans lequel la substance porogène est la mélamine.

2. Procédé selon la revendication 1, dans lequel la zéolite au titane présente une bande d'absorption infrarouge à environ 950 - 960 cm⁻¹.

3. Procédé selon la revendication 1 ou 2, dans lequel la zéolite au titane est une silicalite répondant à la formule xTiO₂(1-x)SiO₂ dans laquelle x est de 0,0001 à 0,5, de préférence de 0,001 à 0,05.

4. Procédé selon l'une quelconque des revendications 1 à 3, dans lequel les granules extrudés sont cylindriques et ont un diamètre de 0,5 à 5 mm, de préférence de 1 à 2 mm, et une longueur de 1 à 8 mm, de préférence de 2 à 4 mm.

5. Procédé selon l'une quelconque des revendications 1 à 4, dans lequel le catalyseur contient de 1 à 99 % en poids, de préférence de 50 à 98 % en poids, de zéolite au titane, le restant étant constitué d'une matrice.

6. Procédé selon l'une quelconque des revendications 1 à 5, dans lequel le plastifiant est un amidon ou une cellulose, de préférence choisie parmi la méthyl-, carboxyméthyl- et hydroxyéthylcellulose.

7. Procédé selon l'une quelconque des revendications 1 à 6, dans lequel la poudre de zéolite au titane mise en oeuvre dans l'étape (a) présente un diamètre moyen inférieur ou égal à 10 µm.

8. Procédé selon l'une quelconque des revendications 1 à 7, dans lequel la quantité de plastifiant mise en oeuvre dans l'étape (a) est d'au moins 1 % et inférieure à 10 % en poids par rapport au poids de zéolite au titane mis en oeuvre.

9. Procédé selon l'une quelconque des revendications 1 à 8, dans lequel la quantité de substance porogène ajoutée au mélange de l'étape (a) est de 6 à 14 % en poids par rapport au poids de zéolite au titane.

## Patentansprüche

1. Verfahren zur Herstellung eines Epoxidierungskatalysators auf der Basis eines Titanzeolithen, der eine ZSM-5-, ZSM-11-, MCM-41-Kristallstruktur aufweist, umfassend:
(a) einen Schritt zum Vermengen eines Gemischs, umfassend ein Titanzeolithpulver, Wasser, mindestens ein Bindemittel in einer Menge von 5 bis 20 Gew.-%, bezogen auf das Gewicht des Titanzeolithen, wobei dieses Bindemittel ausgewählt ist aus den Siloxanen, mindestens einen Weichmacher, ausgewählt aus den Polysacchariden, mindestens einer porogenen Substanz in einer Menge von 5 bis 35 Gew.-%, bezogen auf das Gewicht des Titanzeolithen, und gegebenenfalls anderer Zusatzstoffe, um eine Masse zu bilden,
(b) einen Schritt zum Formen der in Schritt (a) erhaltenen Masse mittels Extrusion, um ein Extrudat zu erhalten,
(c) einen Schritt zum Trocknen, um mindestens einen Teil des Wassers zu entfernen,
(d) einen Schritt zum Kalzinieren, um mindestens einen Teil der vorhandenen organischen Rückstände zu entfernen, um das Bindemittel in einen Stoff umzuwandeln, der die Matrix des Katalysators bildet, und um die porogene Substanz zu entfernen und um so die Porosität des Katalysators zu erhöhen,
und umfassend einen Schritt zur Granulierung, die zwischen Schritt (b) zur Extrusion und Schritt (c) zum Trocknen oder nach Schritt (d) zum Kalzinieren durchgeführt wird, um Extrusionsgranulat zu erhalten, wobei die porogene Substanz Melamin ist.

2. Verfahren nach Anspruch 1, wobei der Titanzeolith eine IR-Absorptionsbande bei etwa 950 bis 960 cm⁻¹ aufweist.

3. Verfahren nach Anspruch 1 oder 2, wobei der Titanzeolith ein Silicalit mit der Formel xTiO₂(1-x)SiO₂ ist, wobei x gleich 0,0001 bis 0,5, bevorzugt 0,001 bis 0,05, ist.

4. Verfahren nach einem der Ansprüche 1 bis 3, wobei das Extrusionsgranulat zylinderförmig ist und einen Durchmesser von 0,5 bis 5 mm, bevorzugt von 1 bis 2 mm, und eine Länge von 1 bis 8 mm, bevorzugt von 2 bis 4 mm, aufweist.

5. Verfahren nach einem der Ansprüche 1 bis 4, wobei der Katalysator 1 bis 99 Gew.-%, bevorzugt 50 bis 98 Gew.-%, Titanzeolith enthält, wobei der Rest aus einer Matrix besteht.

6. Verfahren nach einem der Ansprüche 1 bis 5, wobei der Weichmacher eine Stärke oder eine Cellulose ist, bevorzugt ausgewählt aus Methyl-, Carboxymethyl- und Hydroxyethylcellulose.

7. Verfahren nach einem der Ansprüche 1 bis 6, wobei das Titanzeolithpulver, das in Schritt (a) eingesetzt wird, einen mittleren Durchmesser von kleiner oder gleich 10 µm aufweist.

8. Verfahren nach einem der Ansprüche 1 bis 7, wobei die Menge an Weichmacher, die in Schritt (a) eingesetzt wird, bezogen auf das eingesetzte Titanzeolithgewicht, mindestens 1 Gew.-% und weniger als 10 Gew.-% beträgt.

9. Verfahren nach einem der Ansprüche 1 bis 8, wobei die Menge an porogener Substanz, die dem Gemisch aus Schritt (a) zugefügt wird, bezogen auf das Gewicht des Titanzeolithen, 6 bis 14 Gew.-% beträgt.

## Claims

1. Process for the manufacture of an epoxidation catalyst based on titanium zeolite having a crystalline structure of the ZSM-5, ZSM-11, MCM-41 type, comprising:
(a) a step of blending a mixture comprising a titanium zeolite powder, water, at least one binder in an amount from 5% to 20% by weight of the weight of the titanium zeolite, this binder being chosen from siloxanes, at least one plasticizer chosen from polysaccharides, at least one pore generating substance in an amount of 5 to 35% by weight of the weight of the titanium zeolite, and optionally other additives, in order to form a paste,
(b) a step of shaping the paste obtained in step (a) by extrusion, in order to obtain an extrudate,
(c) a step of drying in order to remove at least some of the water,
(d) a step of calcining in order to remove at least some of the organic residues present, in order to convert the binder into a mateual forming the matrix of the catalyst, and in order to remove the pore generating substance and thereby increase the porosity of the catalyst,
and comprising a granulation step carried out between the extrusion step (b) and the drying step (c) or after the calcining step (d), in order to obtain extruded granules, wherein the pore generating substance is melamine.

2. Process according to Claim 1, wherein the titanium zeolite has an infrared absorption band at about 950 - 960 cm⁻¹.

3. Process according to Claim 1 or 2, wherein the titanium zeolite is a silicalite satisfying the formula xTiO₂(1-x)SiO₂ in which x is from 0.0001 to 0.5, preferably from 0.001 to 0.05.

4. Process according to any one of Claims 1 to 3, wherein the extruded granules are cylinducal and have a diameter of from 0.5 to 5 mm, preferably from 1 to 2 mm, and a length of from 1 to 8 mm, preferably from 2 to 4 mm.

5. Process according to any one of Claims 1 to 4, wherein the catalyst contains from 1 to 99% by weight, preferably from 50 to 98% by weight, of titanium zeolite, the remainder consisting of a matrix.

6. Process according to any one of Claims 1 to 5, wherein the plasticizer is a starch or a cellulose, preferably selected from methyl cellulose, carboxymethyl cellulose and hydroxyethyl cellulose.

7. Process according to any one of Claims 1 to 6, wherein the titanium zeolite powder employed in step (a) has a mean diameter of less than or equal to 10 µm.

8. Process according to any one of Claims 1 to 7, wherein the amount of plasticizer employed in step (a) is at least 1% and is less than 10% by weight relative to the weight of titanium zeolite employed.

9. Process according to any one of Claims 1 to 8, wherein the amount of pore generating substance added to the mixture of step (a) is from 6 to 14% by weight of the weight of the titanium zeolite.
